(19) Europäisches Patentamt
European Patent Office
Office européen des brevets

(11) Publication number: 0 292 171 A2

# (12) EUROPEAN PATENT APPLICATION

(21) Application number: 88304175.8

(22) Date of filing: 09.05.88

(51) Int. Cl.[4]: G01N 33/58 , G01N 33/535 , //G01N33/542,G01N33/94

(30) Priority: 18.05.87 US 50352

(43) Date of publication of application: 23.11.88 Bulletin 88/47

(84) Designated Contracting States: BE CH DE ES FR GB IT LI NL SE

(71) Applicant: TECHNICON INSTRUMENTS CORPORATION
511 Benedict Avenue
Tarrytown, New York 10591(US)

(72) Inventor: Adolfsen, Robert H.
52 Coachlight Square
Montrose New York 10548(US)
Inventor: Saini, Mohan S.
27 Harmony Road
Spring Valley New York 10977(US)
Inventor: Vunnam, Ranga R.
36 Bellwood Drive
New City New York 10956(US)

(74) Representative: Wain, Christopher Paul et al
A.A. THORNTON & CO. Northumberland House 303-306 High Holborn
London WC1V 7LE(GB)

## (54) Enzyme immunoassay.

(57) An analyte in a test sample is analysed by forming a mixture of:
a) the sample containing the analyte under assay;
b) an enzyme capable of mediating a process which yields a detectable signal;
c) a conjugate comprising the same analyte as is under assay, covalently bonded to a reaction intermediate or reaction intermediate analog for the enzyme, the reaction intermediate or analog moiety of said conjugate being capable of transferring an activating group to the active site of the enzyme whereby the process mediated by the enzyme is initiated;
d) a specific binding partner of the analyte, said specific binding partner being capable of binding to said conjugate, whereby said reaction intermediate or analog moiety is restricted from transferring said activating group to the enzyme;
e) the elements of the process mediated by the enzyme;
and measuring the detectable signal produced by the process mediated by the enzyme and therefrom determining the amount of analyte in the sample.

A test composition comprises the enzyme, the conjugate, the binding partner and the signal generating process reagents.

EP 0 292 171 A2

## ENZYME IMMUNOASSAY

This invention relates to an enzyme immunoassay, and to a test composition useful therefor.

Specific binding assays are extremely useful analytical methods for determining various organic substances of diagnostic, medical and environmental importance which appear in liquid media at very low concentration. Specific binding assays are based on the specific interaction between a bindable analyte under determination and a binding partner therefor.

In a conventional, non-isotopic competitive binding assay, a reagent is provided which includes: (1) a labeled conjugate in the form of an analyte which is the same as that to be detected (e.g. an antigen or hapten), which constitutes the binding component of the conjugate, chemically linked to a label component; and (2) a specific binding partner for the analyte (e.g. an antibody). Upon combining the test sample and the reagent, any analyte in the test sample competes with the binding component of the labeled conjugate, in a substantially nondiscriminating manner, for noncovalent binding sites on the specific binding partner. The amount of labeled conjugate bound to the binding partner (i.e. the bound fraction) or the amount of labeled substance which remains unbound to the binding partner (i.e. the free fraction) is a function of the amount of analyte present in the test sample, and the amount of analyte in the test sample can thus be determined by measuring the label in the free or bound fraction.

Where the labeled conjugate in the bound fraction cannot be distinguished from that in the free fraction by the means used to monitor the label component, one fraction must be physically separated from the other in order to complete the assay. This type of assay is referred to in the art as "heterogeneous". Where the bound fraction and free fraction can be distinguished, a "homogeneous" format may be followed and the separation step avoided.

The first discovered type of highly sensitive specific binding assay was the radioimmunoassay which employs a radioactive isotope as the label. Such an assay necessarily must follow the heterogeneous format since the monitorable character of the label is qualitatively unchanged in the free and bound fractions. Because of the inconvenience and difficulty of handling radioactive materials, many new assay systems have been devised using materials other than radioisotopes as the label component, including enzymes, fluorescent molecules, bacteriophages, metals and organometallic complexes, coenzymes, enzyme substrates, enzyme inhibitors, cyclic reactants, and chemiluminescent reactants.

Exemplary of methods which have been developed using an enzyme as the labeling substance are those described in U.S. Patent Nos. 3,654,090; 3,791,932; 3,839,153; 3,850,752; and 3,879,262 and in the Journal of Immunological Methods 1:247 (1972) and the Journal of Immunology 109:129 (1972). In each of the described methods an enzyme is chemically coupled to either the ligand to be detected or a binding partner thereof and an appropriate heterogeneous specific binding reaction scheme is constructed whereby after incubation with a sample, the amount of enzymatic activity associated with either the insoluble portion or the liquid portion is a function of the amount of ligand in the sample. The problems associated with the synthesis, characterization, and stability of the enzyme-conjugates are serious shortcomings of this approach.

An enzyme-labeled immunoassay of the homogeneous type is described in U.S. Patent No. 3,817,834 wherein a ligand enzyme conjugate is employed. The enzymatic activity of the conjugate in the bound fraction is measurably less than that in the free fraction thereby allowing a homogeneous format to be used. The use of particular materials as labeling substances, including chemiluminescent molecules, cyclic reactants, and cleavable fluorescent enzyme substances in both homogeneous and heterogeneous formats is described in German Offenlegungschriften Nos. 2,618,419 and 2,618,511 (Miles Laboratories, Inc.).

British Patent No. 1,392,403 and French Patent No. 2,201,299, which correspond to U.S. Patent No. 3,880,934, describe a specific binding assay which utilizes a non-active percursor of a spectrophotometrically-active substance as the labeling substance. After incubation of the sample with the specific binding reaction system, the insoluble and liquid portions are separated and the amount of labeling substance present in the liquid portion, which is a function of the amount of ligand to be detected in the sample, is determined by carrying out reaction steps that transform the active labeling substance into a chromogen of fluorometrically active material which is then measured by conventional means.

Other specific binding assay methods employing different types of labeling substances are disclosed in: U.S. Patent No. 3,850,578 which discloses the use of electron spin resonance as a labeling means; U.S. Patent No. 3,901,654 which discloses the use of fluorescence quenching and enhancement as a labeling means; and Report No. PB-224,875 of the National Technical Information Service (NTIS) of the U.S. Department of Commerce (1973) which describes an unsuccessful attempt to use hemin chloride as a labeling substance in a heterogeneous assay system monitored by a chemiluminescence reaction. Nature

219:186 (1968) describes in detail certain radioimmunoassay procedures and makes a passing reference of a very general nature to the possible use of coenzymes and viruses in place of radioisotopes as labeling substances. However, the author provides no enlightenment as to how to carry out an assay using such alternative labeling substances, or in fact as to whether such an assay would be operable. For further background, reference may be had to Principles of Competitive Protein-Binding Assays, ed. Odell and Daughaday (J. B. Lippincott Co., Philadelphia, 1972) which discusses in breadth the various known assay schemes and the different materials and features that have been used as labels for specific binding assays.

Even though many new types of specific binding assays have been suggested and investigated, the radioimmunoassay and the various enzyme-tagged immunoassays remain the most widely used and improved. However, both types of systems have obvious shortcomings, the radioimmunoassay in the use of radioactive material which is hazardous and which requires careful handling and the enzyme-tagged immunoassays in the difficulty of preparing useful enzyme-tagged conjugates which remain stable.

U. S. Patent No. 4,134,792 describes a specific binding assay method employing, as a labeling substance, a reversibly binding enzyme modulator that may be used in both homogeneous and heterogeneous binding assay formats wherein the liquid medium to be assayed for a ligand is combined with a reagent comprising a labeled conjugate to form a binding reaction system having a bound-species and a free-species of the conjugate. The distribution of the conjugate between the bound and the free fractions is determined by addition of an enzyme whose activity is affected, either in an inhibitory (e.g. competitive inhibitor) or stimulatory (e.g. allosteric effector) manner, by said modulator and measuring the resulting activity of the enzyme.

U. S. Patent No. 4,273,866 describes a method for determining ligands in test samples comprising intermixing with the test sample a ligand analog-irreversible enzyme inhibitor conjugate and a binding protein bindable to the ligand and the ligand analog-irreversible enzyme inhibitor conjugate and wherein the amount of ligand analog-irreversible enzyme inhibitor conjugate bound by the binding protein is related to nt of ligand in the test sample, said binding protein inactivating the irreversible enzyme inhibitor when bound to the ligand analog portion of the conjugate; intermixing an enzyme which is irreversibly inhibited by the ligand analog-irreversible enzyme inhibitor conjugate unbound by the binding protein; and intermixing substrate to the enzyme and monitoring the enzyme substrate reaction.

U. S. Patent No. 4,230,797 describes a heterogeneous specific binding assay method and means based on the use of a labeling substance and reactant substance which exhibits reactant activity as a constituent of a predetermined reaction system. The amount of the reactant present in either of the bound and free fractions is determined by contacting either fraction with at least one reagent which forms, with the reactant, the predetermined reaction system which serves as a means for monitoring the specific binding reaction. The method requires a separation step of the bound-phase and free-phase.

U. S. Patent No. 4,279,992 describes a method and reagent for determining a ligand in a liquid medium employing, as an enzyme-cleavable substrate label, a residue having the formula

G-D-R

wherein G is a glycone, D is a dye indicator moiety, and R is a linking group through which the label residue is covalently bound to a binding component of a conventional binding assay system, such as the ligand, an analog thereof, or a specific binding partner thereof. The monitored characteristic of the label is the release of a detectable product, usually a fluorogen or chromogen, upon enzymatic cleavage of the glycosidic linkage between the glycone and the dye indicator moiety. The assay method may follow a homogeneous or heterogeneous format.

U. S. Patent No. 4,238,565 describes an assay for determining a ligand in a liquid medium employing an organic prosthetic group residue, such as a residue of flavin adenine dinucleotide, flavin mononucleotide, or heme, as a label component in the labeled conjugate. Preferably, the label component is the prosthetic group residue alone or is a holoenzyme residue comprising such prosthetic group residue combined with an apoenzyme in the form of a holoenzyme complex. In the former case, the label component preferably is monitored in the assay by adding an apoenzyme after the binding reaction has been initiated and measuring the resultant holoenzyme activity. In the latter case, the label component is monitored simply by measuring holoenzyme activity. The assay method may follow conventional homogeneous and heterogeneous schemes.

U. S. Patent No. 4,463,090 describes enzyme immunoassays whose sensitivity is increased by cascade amplification. The coupled ligand (enzyme or an activator) catalytically activates a second enzyme which acts on a substrate or can act on a third enzyme to produce a cascade.

"Zymogen Activation: A New System for Homogeneous Immunoassay" Clin. Chem., 30, pp. 1452-1456

(1984) describes a three-step cascade reaction in which blood clotting Factor X conjugated to an antigen is converted into an active form after a competitive binding process. A peptide is cleaved off the molecule to convert the inactive form into an active form.

In accordance with the present invention, enzyme immunoassays are provided in which an activating group from a reaction intermediate converts an enzyme from an inactive to an active state thereby initiating a detectable signal generating process. The signal produced is a function of the amount of analyte in the test sample.

The invention provides a test composition which includes an enzyme capable of initiating a signal generating process. The activity of the enzyme is modified if an activating group is covalently bonded thereto. A conjugate including a reaction intermediate or a reaction intermediate analog capable of supplying the activating group for the enzyme and the analyte is provided, along with a specific binding partner for the analyte, and reagent means responsive to the condition of the enzyme for generating a detectable signal in proportion to the amount of enzyme activated.

The invention further provides a method of detecting the presence and amount of an analyte in a sample which method consists essentially of the steps of: (1) making a mixture of (a) an enzyme capable of initiating a process which yields a detectable signal when an activating group is covalently bound to its active site; (b) a conjugate comprising an analyte (which is the same as the analyte under assay) covalently bonded to the reaction intermediate or reaction intermediate analog capable of supplying the activating gruop for the enzyme; (c) a specific binding partner of the analyte which is capable of binding to the conjugate and thereby restricting the transfer of the activating group to the enzyme; (d) the elements of the process mediated by the enzyme, and (e) the sample; and (2) measuring the process initiated by the modified enzyme. The analyte in the sample can then be determined by, for example, comparing the rate of the signal generating process conducted in the presence of the sample with the rate of the process when conducted in the presence of a series of standard compositions containing known amounts of the analyte.

The invention also includes a test composition for use in a specific binding assay for detecting an analyte in a sample, said test composition comprising:

(a) an enzyme capable of mediating a signal generating process, the catalytic activity of said enzyme being modifiable by bonding an activating group to the active site of said enzyme;

(b) a conjugate of the same analyte as is to be assayed, covalently bonded to a reaction intermediate or reaction intermediate analog for said enzyme, said reaction intermediate or analog moiety capable of supplying said activating group for said enzyme;

(c) a specific binding partner of the analyte, said specific binding partner being capable of binding to said conjugate, whereby said reaction intermediate or analog moiety is restricted from transferring said activating group to said enzyme; and

(d) reagent means responsive to the condition of said enzyme for generating a detectable signal.

In the accompanying drawing, which is presented to further describe the invention and to assist in its understanding through clarification of its various aspects, the single Figure 1 is a scatter plot of the correlation between the reaction intermediate enzyme immunoassay (RIEIA) of the present invention for theophylline as compared with an assay pursuant to the Technicon Latex Turbidimitry method.

The assays of this invention may be used for the qualitative and/or quantitative measurement of any substance which may be assayed using an antibody-antigen reaction, i.e. an immunoassay such as for example the detection of a drug or hormone in a test sample. In contrast to conventional enzyme-linked immunoassays, the present assay uses a reaction intermediate which supplies an activating group for covalent bonding to an enzyme which converts the enzyme from its inactive to its active state, thereby resulting in the converted enzyme initiating a signal generating process. A novel labeling substance is used which is a conjugate between the same analyte or hapten as is to be measured and the reaction intermediate of an enzyme reaction. As the immunoassay proceeds, the amount of conjugate existing free in solution will depend upon the amount of analyte in the specimen. Free conjugate is detected by the enzyme which is capable of utilizing the free conjugate in place of its normal reaction intermediate from which it obtains an activating group.

Preferred embodiments of the present invention utilize a specific class of convertible enzymes, i.e. phosphomutases. Conjugate which is not bound to antibody in the assay is available to interact with the dephospho form of a phosphomutase, which does not have enzymatic activity. The enzyme abstracts a phosphate group from the conjugate, as a consequence of which it becomes fully active and initiates a detectable signal generating process as described hereafter. The phosphate is bound at the active site of the enzyme, not at a regulatory site. The phosphate in question participates directly in the enzyme reaction, being replaced by a new phosphate from another substrate molecule in each catalytic cycle of the reaction.

In the following discussion, examples of suitable phosphomutases and enzyme activators will be given

followed thereafter by examples illustrative of the present invention.

Suitable phosphomutases are listed in Table 1. Each exists in a phosphorylated form which is active and a dephosphorylated form which is not active, and all have reaction intermediates which are diphosphates. These enzymes are described in W. R. Ray, Jr., and E. J. Peck, Jr. in The Enzymes, 6 407 (1972).

TABLE 1

Some Examples of Phosphomutases

Phosphoglucomutase
Phosphoacetylglucosamine mutase
Phosphomannosamine mutase
Phosphopentomutase
Phosphoglycerate mutase

Illustrative of the present invention is the reaction mechanism for phosphoglucomutase (PGM) which is shown in Scheme I, in which the enzyme complexes are shown below the horizontal line, and substances binding to and dissociating from the enzyme are shown above the line (Cleland's notation). The catalytic cycle begins with the phosphoenzyme (E-P) which is active. After binding with glucose 1-phosphate (G1P), the phosphoenzyme transfers its covalently bound phosphate group to the glucose 1-phosphate substrate to produce the reaction intermediate, glucose 1,6-diphosphate (G16P). The reaction intermediate (G16P) then transfers its 1- phosphate to the enzyme to regenerate E-P and to produce glucose 6-phosphate (G6P), which then dissociates from the enzyme. The cycle then repeats.

SCHEME I

Enzyme Mechanism For Phosphoglucomutase

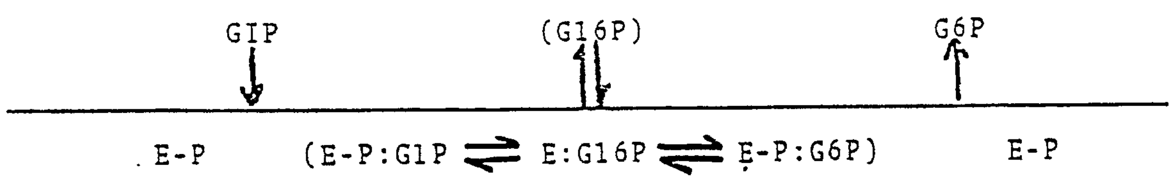


In Scheme I, glucose 1,6-diphosphate (G16P) is the reaction intermediate and it may or may not dissociate during the reaction. Indeed, calculations have shown that this dissociation and reassociation may occur only about one time in twenty (W. R. Ray, Jr., and G. A. Roscelli, J. Biol. Chem., 239, 1228 (1964). This optional dissociation is indicated in Scheme I by enclosing G16P in parantheses. Since the dissociation is not a required step in the sequence, the enzyme mechanism is classified as "uni-uni" in the terminology of enzyme kinetics. That is, the enzyme mechanism is unimolecular with respect to the substrates and unimolecular with respect to products (i.e., one substrate, one product).

Phosphoglucomutase is comprised of a single polypeptide chain with a molecular weight of 65,000. It has no subunits. It has only one active site and it has no regulatory sites. It is very stable and can be stored for years at 4° C as an ammonium sulfate slurry. It has been reported to have a specific activity as high as 900 units/mg, which is equivalent to a turnover number of 65,000 moles of product produced per minute per mole of enzyme. However, the commercially available enzyme (Sigma Co., St. Louis, MO 14508) has a specific activity of only 100-300, this being equivalent to a turnover number of between 7,000 and 20,000. Since the substrate of PGM is anionic, the enzyme is competitively inhibited by a variety of anions, such as chloride.

The three dimensional structure of phosphoglucomutase has recently been determined by x-ray crystallography (Z. Lin, et al., J. Biol. Chem., 261, 264 (1986)). The active site of the enzyme resides at the bottom of a deep crevice. Serine residue 116 in this site is the one which is covalently phosphorylated in E-P. This is the phosphate which is transferred to glucose 1-phosphate to produce the reaction intermediate, glucose 1,6-diphosphate. Subsequently, the 1-phosphate of the reaction intermediate is transferred back to this same serine residue to produce glucose 6-phosphate and to regenerate the phosphoprotein. There are a number of arginine residues located nearby, which may be important for the binding of the phosphate groups of glucose 1,6-diphosphate. In addition, the enzyme has a tightly bound magnesium ion, which interacts with the serine of the dephosphoenzyme and neighboring histidine residues. This magnesium ion greatly facilitates the binding of the substrate glucose 1-phosphate.

Phosphoglucomutase is attractive for use in an immunoassay for two major reasons. First, the active phosphoenzyme has a high turnover number, as high as 65,000, resulting in an immunoassay of high sensitivity. Second, the apparent Km for the reaction intermediate is about $10^{-8}$ M. This would also contribute high sensitivity to the immunoassay. Theoretical calculations suggest that the enzyme should be able to detect free glucose 1,6-diphosphate concentrations as low as $10^{-10}$ M. This prediction has been verified by experimentation.

Some examples of enzyme activators are shown in Table II. The natural reaction intermediate for phosphoglucomutase (PGM) is glucose 1,6-diphosphate. The natural reaction intermediate for phosphoglucosamine mutase (PAGM) is glucosamine 1,6-diphosphate. The natural reaction intermediate for phosphoglycerate mutase is 2,3-diphosphoglycerate. However, a wide variety of other sugar diphosphates can be substituted for the natural reaction intermediate and function as initiators for the reaction. Other similar materials and functional analogs of the listed materials may be able to substitute for the naturally occurring activator enzymes. (For example, a molecule containing one sulfate group and one phosphate group is considered to be substantially equivalent to the similar molecule containing two phosphate groups.)

## TABLE II

## Examples of Enzyme Activators

Glucose 1,6-diphosphate
Glucosamine 1,6-diphosphate
Ribose 1,6-diphosphate
Mannose 1,6-diphosphate
Galactose 1,6-diphosphate
N-acetylglucosamine 1,6-diphosphate
Fructose 1,6diphosphate
2,3-diphosphoglyceric acid

The interactions of some of the activators listed in Table II with PGM have been studied by H. Mulhausen and J. Mendicino, J. Biol. Chem., 245, 4038 (1970) and O. H. Lowry and J. V. Passonneau, J. Biol. Chem., 244, 910 (1969). The interactions of some of these materials with PAGM has been studied by A. Fernandez-Sorensen and D. M. Carlson, J. Biol. Chem., 246, 3485 (1971). The interaction of 2,3-diphosphoglycerate with phosphoglycerate mutase has been studied by P. J. Stankiewicz and L. F. Hass (J. Biol. Chem., 261, 12715 (1986).

In the traditional heterogeneous assay format, an antibody is mixed with a specimen containing the free hapten or other analyte and the conjugate which contains the reaction intermediate covalently attached to analyte. Then a separation of free conjugate from bound conjugate is effected by conventional procedures. Free conjugate is then assayed by incubation with the enzyme, the result of which is a conversion of the enzyme from an inactive state to an active state. The activity of the enzyme is then measured. If all components are soluble, separation of free conjugate may be effected by ultra filtration (for example, using Amicon ultrafiltration protein concentrators or gel filtration columns). Or, an agent may be added which precipitates antibody-analyte and antibody-conjugate complexes, such as anti-antibody. Or, the antibody may be immobilized on a tube or a microliter plate, and the solution containing free conjugate is physically transferred to a separate tube. The incubation with antibody is carried out either in a competitive or sequential manner. In the latter case, the antibody is incubated with the specimen prior to the addition of

the conjugate, which may improve sensitivity somewhat. It should be understood that these are just a few of many possible ways in which heterogeneous assays can be carried out using this concept.

In a homogeneous assay, separation of free and bound conjugate is not necessary. For example, if the antibody restricts bound conjugate from acting as a reaction intermediate for the enzyme, then no separation is required. The assay protocol is either competitive or sequential. In the latter case, the antibody is incubated with the specimen before the conjugate is added, which may improve sensitivity somewhat. Suitable assay formats are described in U.S. Pat. Nos. 4,230,797; 4,134,792 and 4,318,983.

Generally, the enzyme immunoassay in accordance with the present invention involves the use of an enzyme which is capable of initiating a process that yields a detectable signal. By the term "detectable signal" is meant a change in or appearance of a property in the enzyme system which is capable of being perceived or sensed, either by direct observation or instrumentally, and which is a function of the presence of the analyte in the sample. Some examples of detectable signals are changes in visible or infra-red absorption fluorecsence, phosphorescence, reflectance or chemiluminescence. Other examples of detectable signals may be the change in electrochemical properties.

The immunoassay of the present invention includes a conventional competitive binding equilibrium between: (a) the conjugate of a reaction intermediate and the analyte to be detected; and (b) a specific binding partner for the analyte. This binding equilibrium occurs in the presence of an enzyme which is capable of initiating a detectable signal generating process when an activating group is covalently bonded thereto. The resulting detectable signal is then measured and the quantity of analyte in the test sample can be determined.

One preferred embodiment includes: (a) a competitive binding equilibrium involving a conjugate containing glucose 1-6-diphosphate

$$\begin{array}{l} Ab + Ag\text{-}G16P \rightleftharpoons Ab{:}Ag\text{-}G16P \\ + \\ Ag \\ \downarrow\uparrow \\ Ab{:}Ag \end{array}$$

in the presence of phosphoglucomutase (PGM) as the enzyme. As described above, the concentration of free conjugate is controlled by competition between conjugate and analyte in the test sample for binding sites on an antibody. Free conjugate is then scavenged by dephospho-PGM. This enzyme abstracts the 1-phosphate from the conjugate, thereby activating itself. The active enzyme catalyzes a detectable signal generating sequence wherein: (i) phosphoglucomutase produces as a signal precursor glucose 6-phosphate from glucose 1-phosphate; (ii) glucose 6-phosphate dehydrogenase produces the reduced form of nicotinamide adenine dinucleotide (NADH) and 6-phosphate gluconate from glucose 6-phosphate and nicotinamide adenine dinucleotide (NAD); and (iii) the rate of NADH production is then measured.

$$G1P \xrightarrow{PGM} G6P$$

$$G6P + NAD \xrightarrow{G6PDH} 6PG + NADH$$

The rate of appearance of NADH can be monitored at 340 nm in a spectrophotometer. The appearance of NADH may also be monitored by fluorescence spectroscopy or electrochemical means.

An increase in sensitivity can be achieved by adding diaphorase to the scheme. This enzyme utilizes

NADH as a signal precursor, producing a formazan as a signal material which has an extinction coefficient three-fold higher than that of NADH. This reaction is as follows:

$$\text{NADH} + \text{tetrazolium} \xrightarrow{\text{diaphorase}} \text{NAD} + \text{formazan}$$

A similar process can be effected using chemical mediators such as phenazine methosulfate in place of diaphorase.

Additional sensitivity can also be obtained by adding 6-phosphogluconic acid dehydrogenase (6PGDH) to the system. This enzyme utilizes 6PG, converting it to ribulose 5-phosphate (R5P), while producing another mole of NADH in the process:

$$\text{6PG} + \text{NAD} \xrightarrow{\text{6PGDH}} \text{R5P} + \text{NADH}$$

It may also be necessary to include a lacontase to convert the δ-lactone of 6PG to 6PG, since the 6PG produced by G6PDH is actually in the form of the δ-lactone, and since 6PGDH does not utilize the δ-lactone very well as a substrate.

An additional means of further increasing sensitivity is to utilize the NADH as a signal precursor in conjunction with the enzymes FMN oxidoreductase and bacterial luciferase. The improved sensitivity in this case is attributable to the high sensitivity of luminescent/bioluminescent instrumentation.

An additional preferred embodiment of the assay of the present invention utilizes phosphoacetylglucosamine mutase (PAGM) as the phosphomutase. The conjugate contains glucosamine 1,6-diphosphate (or a functionally analogous substance) covalently attached to an antigen. The competitive binding process and the scavenging of free conjugate by the dephospho enzyme are substantially equivalent to that described above for the case of phosphoglucomutase. In this embodiment, the enzyme mediates the conversion of glucosamine 1-phosphate to glucosamine 6-phosphate. It can also mediate the conversion of glucose 1-phosphate to glucose 6-phosphate, as in the case of phosphoglucomutase above. As such, the conversion of signal precursor into signal generating material is also substantially equivalent to that for the previous embodiment.

A further preferred embodiment utilizes phosphomannosamine mutase as the enzyme. In this case the conjugate contains 1,6-diphosphomannosamine (or a functionally analogous substance) covalently attached to the antigen. The competitive binding process and the scavenging of free conjugate by the phosphomannosamine mutase are substantially equivalent to that described above for phosphoglucomutase. This enzyme mediates the conversion of mannosamine 1-phosphate to mannosamine 6-phosphate. It can also mediate the conversion of glucose 1-phosphate to glucose 6-phosphate, as described above for phosphoglucomutase. Therefore, the conversion of signal precursor into signal by coupling enzymes can be equivalent to that described above for phosphoglucomutase.

A further embodiment utilizes pentophosphomutase (PPM). In this case the conjugate contains ribose 1,5-diphosphate (or a functionally analogous substance) covalently attached to the analyte. After competitive binding and scavenging of free conjugate by dephosphoenzyme, the activated enzyme mediates the following process, in which ribose 5-phosphate (R5P) is a signal precursor:

$$\text{ribose 1-phosphate} \xrightarrow{\text{PPM}} \text{ribose 5-phosphate}$$

The incorporation of two additional enzymes are required to convert the signal precursor into the signal generating substance. These enzymes are ribose phosphate pyrophosphokinase (RPPK) and AMP deaminase:

$$R5P + ATP \xrightarrow{RPPK} \text{5-phosphoribosyl 1-pyrophosphate} + AMP$$

$$AMP \xrightarrow{\text{AMP deaminase}} IMP + NH_4^+$$

where AMP is adenosine monophosphate and IMP is inosine monophosphate. The production of ammonium ions by this process may be monitored with a specific ion electrode. Alternatively, one additional enzyme could be used to couple the reaction to a change in absorbance, this enzyme being glutamate dehydrogenase (G1DH):

$$NH_4^+ + \alpha\text{-ketoglutarate} + NADH \xrightarrow{G1DH} \text{glutamate} + NAD$$

An other embodiment utilizes phosphoglyceromutase (PG1yM). In this case the conjugate contains 2,3-diphosphoglyceric acid (or similar substance) covalently attached to the analyte. After competitive binding and scavenging of free conjugate by dephosphoenzyme, the activated enzyme mediates the following process, in which 3-phosphoglyceric acid (3PG) is the substrate and 2-phosphoglyceric acid (2PG) is the signal precursor:

$$3PG \xrightarrow{PG1yM} 2PG$$

The incorporation of three additional enzymes is required to convert the signal precursor into the signal generating substance:

$$2PG \xrightarrow{\text{enolase}} PEP$$

$$PEP + ADP \xrightarrow{\text{pyruvate kinase}} \text{pyruvate} + ATP$$

$$\text{pyruvate} + NAD \xrightarrow{LDH} \text{lactate} + NADH$$

in which PEP is phosphoenolpyruvate and LDH is lactate dehydrogenase. Alternately, ATP could be used as a signal precursor in conjuction with firefly luciferase to produce light, in a bioluminescent reaction which has very high sensitivity.

Another preferred embodiment utilizes two different phosphomutases as a pair to perform an assay in which the best properties of both enzymes may be exploited to produce an assay of very high sensitivity. In this case, the conjugate contains glucosamine 1,6-diphosphate which is covalently attached to the analyte of interest. After the competitive binding process, phosphoacetylglucosamine mutase (E1) is utilized to efficiently scavenge free conjugate:

$$G16P\text{-}Ag + E1 \longrightarrow G6P\text{-}Ag + E1\text{-}P$$

In the presence of glucose 1-phosphate, E1-P equilibrates with glucose 1,6-diphosphate in the active site of the enzyme. This bound reaction intermediate subsequently equilibrates between bound and free states:

$$E1P + G1P \rightleftharpoons E1\text{-}P{:}G1P \rightleftharpoons E1{:}G16P \rightleftharpoons E1 + G16P$$

The second phosphotmutase (E2) is phosphoglucomutase. E2 binds the free reaction intermediate with a high affinity (Ka about $10^8$), thereby providing higher sensitivity to the assay. The following reaction results in production of most of the signal precursor, with smaller amounts being generating by E1:

$$G1P \xrightarrow[G16P]{E2} G6P$$

The subsequent reactions are the same as those described above for phosphoglucomutase alone.

The following working examples describe experiments which were performed in developing the present invention. Standard commercially available reagent grade chemicals were used whenever possible. These examples are to be considered illustrative of the present invention and should not be interpreted as limiting its scope.

## Example 1

### Preparation of Glucosamine 1, 6-Diphosphate

Glucosamine 1,6-diphosphate, a key intermediate required for conjugate synthesis, was synthesized by an enzyme exchange reaction and purified by Dowex chromatography. The method for synthesis of this material was adapted from a method published for the synthesis of N-acetylglucosamine 1,6-diphosphate P. Cheng and D. M. Carlson, Anal. Biochem., 85, 533-540 (1978).

The reaction mixture contained the following components: 1 mM glucose 1,6-diphosphate, 5 mM glucosamine 6-phosphate, 2 mM nicotinamide adenine dinucleotide (NAD), glucose 6-phosphate dehydrogenase (G6PDH) at a concentration of 1 unit/ml, 1 mM magnesium acetate, 0.1 mM ethylene diamine tetracetate (EDTA) and 50 mM Tricine buffer at pH 9.0. The reaction mixture was incubated at 37° C for 30 minutes, at which time the formation of glucosamine 1,6-diphosphate had gone to completion.

Glucosamine 1,6-diphosphate (300 µmole) was purified from the above reaction mixture by chromatography on a column of Dowex 1-$Cl^-$ (dimensions: 1.5 cm x 34 cm). After applying the sample and washing out non-absorbed materials with deionized water, the elution of absorbed materials was carried out using 10 mM HCl. NAD/NADH eluted first, followed by glucosamine 6-phosphate, followed by glucosamine 1,6-diphosphate. NAD/NADH elution was followed by absorption at 260 nm. The elution of glucosamine 6-phosphate and glucosamine 1,6-diphosphate was followed by the ninhydrin method, and the latter was also followed by the ability of this compound to activate phosphoglucomutase. Enzyme activity was determined in a reaction mixture containing 0.2 mM glucose 1-phosphate, 1 mM NAD, 1 mM magnesium acetate, 0.1 mM EDTA, G6PDH at 1 U/ml and 50 mM imidazole-chloride, pH 7.5.

## Example 2

### Preparation of a Theophylline Conjugate

The first step in this synthesis was the preparation of carboxypropyltheophylline. This was done using glutaric anhydride and 5,6-diamino-1,3-dimethyluracil, as reported by Cook, et al. (Res. Common. Chem.

Path. Pharmacol, 13, 497 (1976) .

The second step was the synthesis of the N-hydroxysuccinimide ester of carboxypropyltheophylline. Carboxypropyltheophylline (372 mg, 1.4 mmole) and N-hydroxysuccinimide (NHS) (164 mg, 1.42 mmole) were dissolved in 14 ml of cold, dry dimethylformamide. To this was added 1-ethyl-3-(3-dimethylaminopropyl)-carbodimide (EDC) (314 mg, 1.58 mmole), and the solution was stirred at 4° C overnight.

Glucosamine 1,6-diphosphate, lithium salt (2.2 mg, 6 umoles) was dissolved in 225 µl of water, and 10 µl of triethylamine was added to raise the pH to about 10. This solution was cooled at 4° C and treated with 0.45 ml (45 µmoles) of the NHS ester of carboxypropyltheophylline. This solution was stirred for 18 hours at 4° C. Then 1 ml of 10% sodium carbonate was added to the reaction mixture, followed by 1 ml of additional succinimide ester (100 µmoles), and the stirring was continued overnight at 4° C, followed by three hours more at room temperature. The reaction mixture was then diluted with five ml of water and centrifuged to remove a precipitate.

The conjugate was partially purified by Dowex 1-$Cl^-$ anion exchange chromatography. The Dowex 1-$Cl^-$ was washed three times with 200 mM lithium chloride containing 50% ethanol. This was the eluant which would subsequently desorb the conjugate from the column. The column was then packed, rinsed extensively with deionized water, and the sample was applied. Washing with water continued until no further material absorbing at 275 nm eluted. Stepwise elution with lithium chloride of increasing concentration was used to elute various materials from the column (100 ml each of 50 to 200 mM lithium chloride in steps of 25 mM). Elution with 200 mM lithium chloride was continued until no more material absorbing at 275 nm eluted from the column. Then 200 mM lithium chloride containing 50% ethanol was passed through the column to elute the conjugate. The elution of conjugate was followed both by absorbance at 275 nm and by its activity with phosphoacetylglucosamine mutase (PAGM). The ethanol was removed from the conjugate solution at 37° C under a stream of nitrogen.

Final purification of the conjugate was carried out using high performance liquid chromatogrpahy (HPLC). This was done on a Varian Model 5000 liquid chromatograph, using a Supelco reverse phase LC-8 column (No. 5-8220) with a Supelco reverse phase guard column (No. 5-905Z), a Technicon FAST-LC variable wavelength HPLC detector set at 275 nm, and Spectra-Physics Data Handler Integrator (Model SP-4100). The mobile phase was 0.1 M imidazole acetate buffer, pH 7.0, containing 5% methanol. A 200 µl aliquot of conjugate was injected into the system, and fractions were collected for 45 minutes with a flow rate of 1.5 ml/min. Enzyme activity was used to determine which peak contained the conjugate. It was believed to be a homogeneous compound at this stage. It was stored at -20° C.

The molecular structure of the conjugate is believed to be the following:

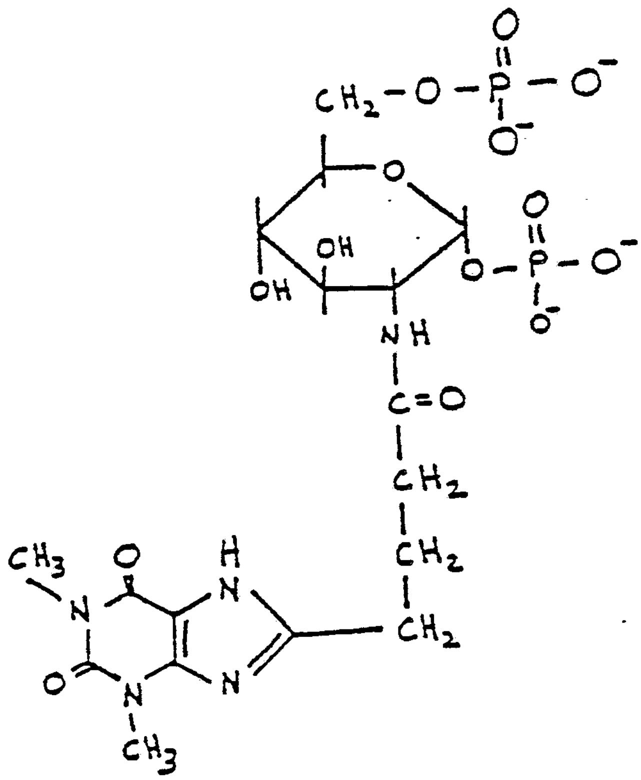

Example 3

Isolation of Phosphoacetylglucosamine Mutase

For the assay to perform in the intended manner in a homogeneous format, the phosphomutase must be able to abstract the 1-phosphate from the conjugate, thereby activating itself. Phosphoacetylglucosamine mutase (PAGM) was used for this purpose. Since this enzyme was not commercially available, it is necessary to isolate it.

A procedure related to Sorensen et al. J. Biol. Chem., 246, pp. 3485-3493 was developed with the objective of inhibiting proteases during the isolation of the enzyme and separating them completely from PAGM. Also, the presence of mucins in the crude extract tended to clog columns. Therefore, Trisacryl ion exchangers were used due to their superior flow characteristics.

Porcine submaxillary glands (500 g) obtained from Per-Freez (Rogers, AR) were thawed at 4°C for 2-3 hours, then manually minced and placed into a beaker containing two liters of 5 mM Tris-HCl (pH 7.5), 10 mM magnesium chloride, 0.2 mM EDTA and 2 mM p-aminobenzamidine (protease inhibitor). The solution was gently shaken for 12 hours at 4°C to extract the enzyme. Tissue fragments were removed by centrifugation, and the supernatant was filtered through cheese cloth. This crude extract was fractionated using ammonium sulfate. The 30-70% fraction contained the enzyme. The precipitate was redissolved in the same buffer described above, containing 4 mM p-aminobenzamidine, and dialysed against more of this same buffer.

The enzyme was purified further by chromatography on a DEAE Trisacryl column (3x28 cm), which was preequilibrated in the same buffer as above, but without the p-aminobenzamidine. The enzyme was eluted in the 0.2 Tris-HCl (pH 7.5) containing 10 mM $MgCl_2$ and 0.2 mM EDTA. The enzyme was concentrated by ammonium sulfate precipitation, then dissolved and dialyzed against 20 mM imidazole acetate (pH 7.5) containing 5 mM magnesium acetate, 0.2 mM EDTA and 1 mM p-aminobenzamidine. The pH was adjusted

to 5.4 with cold 1 M acetic acid just before its application to the CM column (see below).

The enzyme was further chromatographed on a CM Trisacryl column (2x40 cm). The column was preequilibrated in 5 mM Trisacetate (pH 5.0), containing 10 mM $MgCl_2$ and 0.2 mM EDTA. After applying the enzyme, the column was washed with 5 mM Trisacetate, 25 mM imidazole acetate (pH 6.0), containing 0.1 mM EDTA and 1 mM p-aminobenzamidine until the absorbance at 280 nm was less than 0.05. Then PAGM was eluted in 50 mM imidazole acetate pH acetate (pH 7.2), containing 20 mM magnesium acetate, 0.1 mM EDTA and 1 mM p-aminobenzamidine. The fractions containing enzyme activity were pooled, and enzyme was concentrated by ammonium sulfate precipitation and then dialized against 50 mM imidazole acetate (pH 7.5), containing 10 mM magnesium acetate and 1 mM EDTA.

The enzyme was further purified on an S-200 Sephacryl column (100 cm x 1.6 cm). The column was pre-equilibrated with 50 mM imidazole acetate (pH 7.5), 10 mM magnesium acetate and 1 mM EDTA. The enzyme was applied and then eluted with the same buffer. The active fractions were pooled and then concentrated by precipitation in 75% ammonium sulfate. The precipitate was collected by centrifugation and then dialyzed against 25 mM imidazole acetate (pH 7.5), 5 mM magnesium acetate and 0.2 mM EDTA.

The purified enzyme was nearly homogeneous by polyacrylamide gel electrophoresis. The yield was about 500 mg of protein with a specific activity of about 6 umoles/min/mg. It was stable for months at either 4°C or -20°C in the presence of 20% glycerol.

## Example 4

### Reaction Mixture Composition For Homogeneous Assay

One of the most advantageous features of using phosphoglucomutase (PGM) in an immunoassay is the high affinity that this enzyme has for its reaction intermediate, glucose 1,6-diphosphate. The apparent Km is as low at $10^{-8}$ (W. R. Ray, Jr. and E. J. Peck, Jr., The Enzymes, 6, 407-477 (1982). When this Km value was taken as an approximation of the dissociation constant, then theoretical calculations indicated that this system would have a sensitivity limit of about $10^{-10}$ M glucose 1,6-diphosphate. However, to achieve this level of sensitivity in a coupled enzyme assay with G6PDH, substantial optimization was required.

The optimized reaction mixture has the following composition: Glucose 1-phosphate (the substrate for PGM) was optimal at 0.2 mM; higher concentrations were inhibitory. PGM was present at 1000 U/L; this concentrations was necessary to insure that most of the reaction intermediate was enzyme-bound, so that it could express maximal activity. Magnesium acetate was optimal at 1 mM, and 0.1 mM EDTA was present to chelate any heavy metals which might be present. NAD was present at 1 mM. G6PDH was present at 1000 U/L. The G6PDH was from Leuconostoc mesenteroides, so that NAD could be used as a substrate instead of NADP. NADP inhibits PGM, as do many other organic and inorganic anions. The buffer was 20 mM imidazole acetate, pH 7.5. Under these conditions, glucose 1,6-diphosphate at a concentration of $10^{-10}$M gave a signal of 10 mA/min.

For the immunoassay, glucose 1,6-diphosphate was replaced by theophylline conjugate at a concentration of $10^{-7}$M. Also, the PAGM concentration was 50 U/L, and the PGM concentration was 200 U/L.

## Example 6

### Immunoreactivity of The Theophylline Conjugate

For the assay principle to function properly in a homogeneous format, PAGM must be able to utilize free conjugate as an activator, and it must not be able to utilize antibody-bound conjugate as an activator. The results in Table III show that antibody inhibited the ability of PAGM to scavenge conjugate in the desired manner. The antibody was a monoclonal from Beckman (Irvine, CA), clone TAs4 (Part No. 584025).

The reaction mixture was essentially the same as that described in Example 4, with theophylline conjugate at $10^{-7}$M, PAGM at 50 U/L and PGM at 200 U/L. Inhibition did not occur when glucose 1,6-diphosphate was substituted for the conjugate, indicating that the inhibition was immunospecific, requiring the theophylline moiety in the conjugate.

Table III

| Antibody Added (µl) | Activity (mA/min) |
|---|---|
| 0 | 354 |
| 1 | 270 |
| 2 | 204 |
| 4 | 122 |
| 7 | 86 |
| 10 | 69 |

Example 7

Dose Response Curve For Theophylline

The concentration of theophylline was varied to demonstrate the dose response, using the same reaction mixture as in Example 6, with 7.5 µl of antibody per ml of reaction mixture. The serum sample, containing theophylline from 0-40 µg/ml, was diluted 100-fold into the reaction mixture. The results are shown in Table 4. They demonstrate that the competitive binding process occurred in the desired manner.

TABLE IV

| Theophylline (µg/ml) | Activity (mA/min) |
|---|---|
| 0 | 83 |
| 2.5 | 91 |
| 5 | 105 |
| 10 | 145 |
| 20 | 201 |
| 40 | 250 |

Higher sensitivity has been achieved by the incorporation of diaphorase and iodonitrotetrazolium (INT) into the reaction mixture. The diaphorase was from Bacillus stearothermophilus, and it was obtained from Chemical Dynamics Corp. (South Plainfield, NJ). At a concentration of 400 U/L, with INT at 0.5 mM, and 0.1% Triton X-100 in the reaction mixture, the sensitivity was higher by a factor of 2.7.

Example 8

Serum Correlation Study

Using essentially the same reaction mixture as that used in the previous two examples, a number of clinical specimens containing unknown levels of theophylline were tested. The rates were measured, and the theophylline values were read from the standard curve. When the assay was run on the TECHNICON RA-1000 clinical analysis system (TECHNICON RA-1000 is a registered trademark of Technicon Instruments Corporation, Tarrytown, N.Y.) measuring the rate from 1-3 minutes, and when the method for comparison was the Technicon Latex Turbidimitry method, the correlation coefficient was 0.994, the standard error was 0.6 µg/ml, the slope was 0.985, and the intercept was 0.5 µg/ml (see Fig 1).

Similar results were obtained when RIEIA was compared to other immunoassay methods. When correlated to the EMIT method (Syva Co, Palo Alto, CA 94303), the slope was 1.01, the intercept was 1.5 µg/ml, the correlation coefficient was 0.989, and the standard error was 0.9 µg/ml. When correlated to the SLFIA method (Ames Division, Miles Laboratories, Elkhart, IN 46515), the slope was 1.05, the intercept was -0.3 µg/ml, the correlation coefficient was 0.977, and the standard error was 1.6 µg/ml.

These results demonstrated the clinical utility of the method utilizing the immunoassay of the present invention.

It should be understood by those skilled in the art that various modifications may be made in the present invention without departing from the spirit and scope thereof as described in the specification and defined in the amended claims.

## Claims

1. A method of detecting the presence and amount of an analyte in a test sample, comprising forming a mixture of:
a) the sample containing the analyte under assay;
b) an enzyme capable of mediating a process which yields a detectable signal;
c) a conjugate comprising the same analyte as is under assay, covalently bonded to a reaction intermediate or reaction intermediate analog for the enzyme, the reaction intermediate or analog moiety of said conjugate being capable of transferring an activating group to the active site of the enzyme whereby the process mediated by the enzyme is initiated;
d) a specific binding partner of the analyte, said specific binding partner being capable of binding to said conjugate, whereby said reaction intermediate or analog moiety is restricted from transferring said activating group to the enzyme;
e) the elements of the process mediated by the enzyme;
and measuring the detectable signal produced by the process mediated by the enzyme and therefrom determining the amount of analyte in the sample.

2. A method according to claim 1, wherein the activating group becomes covalently bonded to the active site of the enzyme.

3. A method according to claim 1 or 2, wherein said enzyme is a phosphomutase.

4. A method according to claim 3, wherein said enzyme and reaction intermediate are chosen from: phosphoglucomutase and glucose 1,6-diphosphate, phosphoacetylglucosamine mutase and glucosamine 1,6-diphosphate, phosphomannosamine mutase and 1,6-diphosphomannosamine, pentophosphomutase and ribose 1,5-diphosphate, or phosphoglyceromutase and 2,3-diphosphoglyceric acid.

5. A method according to claim 1,2,3 or 4, wherein a second enzyme is included in the mixture to enhance the sensitivity of the detectable signal generating process.

6. A method according to claim 5, wherein said enzyme is phosphoacetylglucosamine mutase and said second enzyme is phosphoglucomutase.

7. A test composition for use in a specific binding assay for detecting an analyte in a sample, said test composition comprising:
a) an enzyme capable of mediating a signal generating process, the catalytic activity of said enzyme being modifiable by bonding an activating group to the active site of said enzyme;
b) a conjugate of the same analyte as is to be assayed, covalently bonded to a reaction intermediate or reaction intermediate analog for said enzyme, said reaction intermediate or analog moiety capable of supplying said activating group for said enzyme;
c) a specific binding partner of the analyte, said specific binding partner being capable of binding to said conjugate, whereby said reaction intermediate or analog moiety is restricted from transferring said activating group to said enzyme; and
d) reagent means responsive to the condition of said enzyme for generating a detectable signal.

8. A test composition according to claim 7, wherein said enzyme and reaction intermediate are selected from: phosphoglucomutase and glucose 1,6-disphosphate, phosphoacetylglucosamine mutase and glucosamine 1,6-diphosphate, phosphomannosamine mutase and 1,6-diphosphomannosamine, pentophosphomutase and ribose 1,5-diphosphate, or phosphoglyceromutase and 2,3-diphosphoglyceric acid.

9. A test composition according to claim 7, wherein said enzyme if phosphoglucomutase.

10. A test composition according to claim 7,8 or 9, further including a second enzyme which enhances the sensitivity of the detectable signal generating process.

11. A test composition according to claim 10, wherein said enzyme is phosphoacetylglucosamine mutase and said second enzyme is phosphoglucomutase, and said reaction intermediate is preferably glucose 1,6-diphosphate.

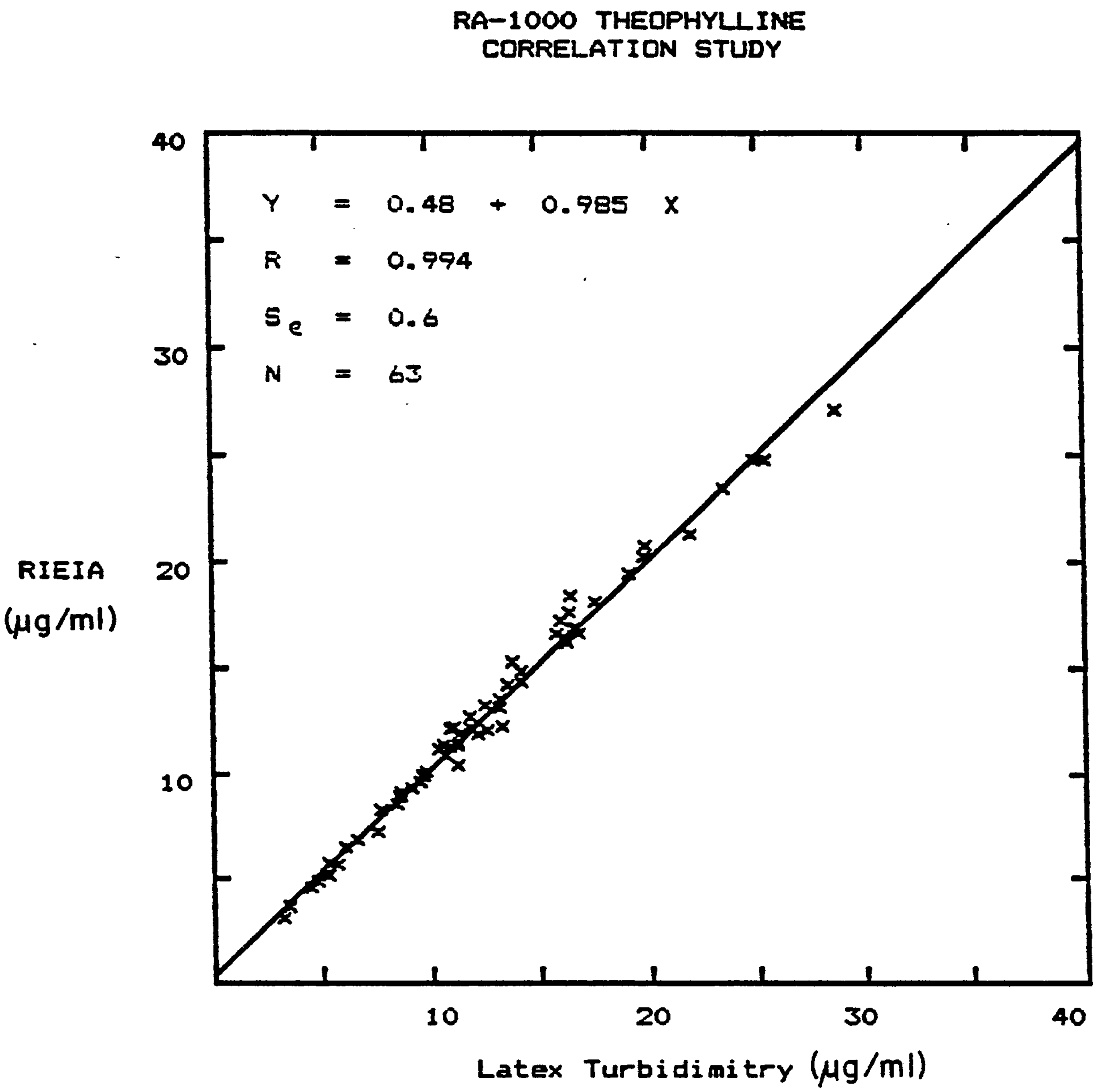

RA-1000 THEOPHYLLINE
CORRELATION STUDY
Y = 0.48 + 0.985 X
R = 0.994
Se = 0.6
N = 63
40
30
20
10
RIEIA
(µg/ml)
10
20
30
40
Latex Turbidimitry (µg/ml)


FIG. 1